(19) Europäisches Patentamt
European Patent Office
Office européen des brevets

(11) Publication number: **0 493 813 A1**

# (12) EUROPEAN PATENT APPLICATION

(21) Application number: **91122308.9**

(51) Int. Cl.[5]: **C07D 241/08**

(22) Date of filing: **27.12.91**

(30) Priority: **27.12.90 JP 418593/90**

(43) Date of publication of application:
**08.07.92 Bulletin 92/28**

(84) Designated Contracting States:
**BE DE FR GB NL**

(71) Applicant: **Ajinomoto Co., Ltd.**
**15-1, Kyobashi 1-chome, Chuo-ku**
**Tokyo(JP)**

(72) Inventor: **Kato, Toshihisa, c/o Central Research Lab.**
**Ajinomoto Co., Inc., 1-1, Suzuki-cho, Kawasaki-ku**
**Kawasaki-shi, Kanagawa-ken(JP)**
Inventor: **Takemoto, Tadashi, c/o Central Research Lab.**
**Ajinomoto Co., Inc., 1-1, Suzuki-cho, Kawasaki-ku**
**Kawasaki-shi, Kanagawa-ken(JP)**

(74) Representative: **Strehl, Schübel-Hopf, Groening**
**Maximilianstrasse 54 Postfach 22 14 55**
**W-8000 München 22(DE)**

## (54) 2,5-Dioxopiperazine compound and processes for preparation thereof.

(57) 2,5-Dioxopiperazine-3-acetamide is prepared by reacting a dialkyl-L-aspartate with monochloroacetic acid, monochloroacetyl chloride or monochloroacetic acid anhydride, and then treating the resulting dialkyl chloroacetyl-L-aspartate with ammonia.

EP 0 493 813 A1

## BACKGROUND OF THE INVENTION

### Field of the Invention

The present invention relates to a process for preparing 2,5-dioxopiperazine-3-acetamide which is an intermediate for the preparation of α-L-aspartyl-L-phenylalanine methyl ester.

### Description of the Background

α-L-Aspartyl-L-phenylalanine methyl ester (α-APM) is in increasing demand as a low calorie sweetener of good quality. A variety of processes for preparing α-APM are already known and one of them requires the contact of 3-benzyl-6-carboxymethyl-2,5-dioxopiperazine or its derivatives with a strong acid in methanol solvent to form α-L-aspartyl-L-phenylalanine dimethyl ester followed by conversion of the dimethyl ester into α-APM (Japanese Patent Application Laid-Open No. 62-183199). However, the only known methods of preparing 3-benzyl-6-carboxymethyl-2,5-dioxopiperazine and its derivatives have also resulted in the by-production of α-APM. A need therefore continues to exist for a method of selectively synthesizing 3-benzyl-6-carboxymethyl-2,5-dioxopiperazine on an industrial scale.

## SUMMARY OF THE INVENTION

Accordingly, one object of the present invention is to prepare 2,5-dioxopiperazine-3-acetamide, which is an intermediate for the preparation of 3-benzyl-6-carboxymethyl-2,5-dioxopiperazine or its derivatives, efficiently on an industrial scale.

Briefly, this object and other objects of the present invention as hereinafter will become more readily apparent can be attained by a method of preparing 2,5-dioxopi perazine-3-acetamide by reacting a dialkyl L-aspartate with monochloroacetic acid, monochloroacetyl chloride or monochloroacetic acid anhydride, and treating the resulting dialkyl chloroacetyl-γ-aspartate with ammonia.

## DETAILED DESCRIPTION OF THE PREFERRED EMBODIMENTS

As a result of extensive investigations for a process by which 2,5-dioxopiperazine-3-acetamide represented by the following formula:

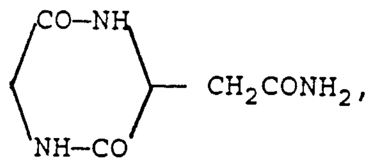

which is an intermediate for the preparation of 3-benzyl-6-carboxymethyl-2,5-dioxopiperazine or its derivatives, can be prepared, it has now been found that the intermediate can be prepared from the inexpensive raw materials monochloroacetic acid and its derivatives and dialkyl -L-aspartates.

2,5-Dioxopiperazine-3-acetamide can be prepared by the following processes:

1) A process which uses monochloroacetic acid as a raw material.

Monochloroacetic acid is condensed with dialkyl L-aspartate in an organic solvent in the presence of a condensing agent such as dicyclohexylcarbodiimide. Upon contact of the resulting dialkyl monochloroacetyl-L-aspartate with ammonia, the desired 2,5-dioxopiperazine-3-acetamide is obtained. The solvent used for the condensation is not particularly limited, as long as it is inert to the raw material, the condensing agent and the product. Suitable preferred examples of the solvent include chloroform, methylene chloride, dioxane, toluene, dimethylformamide, and the like. Suitable condensing agents generally used for peptide synthesis may be used, in addition to dicyclohexylcarbodiimide. Ammonia in the form of ammonia water is most preferred since it is easily accessible. Like ammonia-methanol, the ammonia may be in such a state that ammonia is dissolved in an organic solvent.

2) A process which uses monochloroacetyl chloride as a reactant.

Monochloroacetyl chloride is reacted with dialkyl L-aspartate in an organic solvent in the presence of a base such as triethylamine, or the like. The resulting dialkyl monochloroacetyl-L-aspartate is treated with ammonia as in 1) to give 2,5-dioxopiperazine-3-acetamide. The solvent used for the condensation is not particularly limited, as long as it is inert to the raw material, the condensing agent and the product. Suitable preferred examples of the solvent include chloroform, methylene chloride, dioxane, toluene, dimethylformamide, and the like. Suitable bases include organic amines such as triethylamine, methylmorpholine, and the like, which are preferred, but an aqueous solution of an inorganic base such as sodium carbonate, sodium hydrogencarbonate, or the like may also be used.

3) A process which uses monochloroacetic acid anhydride as a reactant.

Monochloroacetic acid anhydride is reacted with dialkyl L-aspartate in an organic solvent in the presence of a base such as triethylamine, or the like. The resulting dialkyl monochloroacetyl-L-aspartate is treated with ammonia as in 1) to give 2,5-dioxopiperazine-3-acetamide. The solvent used for the condensation is not particularly limited as long as it is inert to the raw material, the condensing agent and the product. Suitable preferred examples of the solvent include chloroform, methylene chloride, dioxane, toluene, dimethylformamide, and the like. Suitable bases include organic amines such as triethylamine, methylmorpholine, and the like, which are preferred, but an aqueous solution of inorganic bases such as sodium carbonate, sodium hydrogencarbonate, or the like may also be used.

The reaction of the dialkyl monochloroacetyl-L-aspartate with ammonia proceeds as follows to give the desired 2,5-dioxopiperazine-3-acetamide.

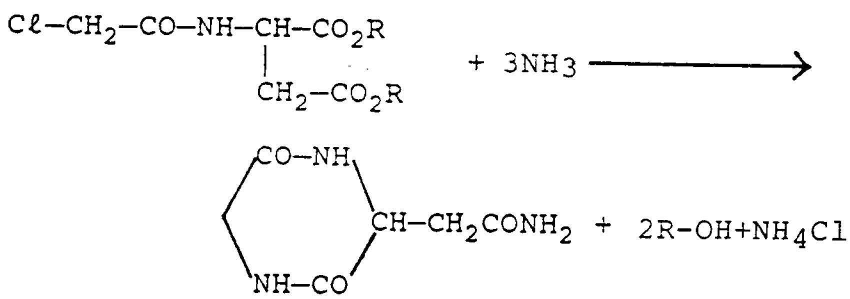

wherein R is alkyl.

The amount of ammonia employed in the reaction may be 2-fold moles or more, preferably 2 to 100-fold moles, based on dimethyl monochloroacetyl-L-aspartate. The reaction temperature is not particularly limited.

The alkyl moiety of the dialkyl monochloroacetyl-L-aspartate may be methyl, ethyl, propyl, isopropyl, butyl, or the like. In view of such factors as cost, ease of esterification, and the like, methyl and ethyl are preferably used.

Having now generally described this invention, a further understanding can be obtained by reference to certain specific examples which are provided herein for purposes of illustration only and are not intended to be limiting unless otherwise specified.

Example 1

To 150 ml of chloroform solution containing 16.1 g (0.1 mol) of dimethyl L-aspartate was added 9.5 g (0.1 mol) of monochloroacetic acid. The mixture was stirred. While cooling the solution with ice, 21.7 g (0.105 mol) of dicyclohexylcarbodiimide was added to the mixture followed by stirring for an hour. Thereafter the mixture was stirred at room temperature overnight. The precipitated dicyclohexylurea was removed by filtration. The filtrate was concentrated under reduced pressure and the residue was dissolved in 150 ml of ethyl acetate. After washing successively with 2N-HCl, 5% $NaHCO_3$ solution and saturated aqueous sodium chloride solution, the ethyl acetate layer was dried over anhydrous sodium sulfate. Sodium sulfate was removed by filtration and the filtrate was concentrated under reduced pressure. To the residue was added 40 ml of 28% ammonia water. The mixture was stirred at room temperature for 24 hours. The precipitated crystals were separated by filtration, washed with cooled water and then dried to give 7.4 g (0.043 mol) of 2,5-dioxopiperazine-3-acetamide. m.p. 263-265 °C. The yield was 43% based on dimethyl L-

aspartate.

$^1$H NMR ($D_2O$) δ 2.87 (m, 2H) , δ 405 (m, 2H) , δ 4.39 (m, 1H) MS (FAB) 172 ($MH^+$)

Example 2

While stirring at ice cooled temperatures, 50 ml of toluene solution containing 11.4 g (0.1 mol) of monochloroacetyl chloride was added to 150 ml of toluene solution containing 16.1 g (0.1 mol) of dimethyl L-aspartate and 14 ml (0.1 mol) of triethylamine. After stirring for an hour at ice cooled temperature and for 3 hours at room temperature, the mixture was washed successively with 2N-HCl, 5% $NaHCO_3$ solution and saturated aqueous sodium chloride solution and then finally dried over anhydrous sodium sulfate. Sodium sulfate was removed by filtration and the filtrate was concentrated under reduced pressure. To the residue was added 40 ml of 28% ammonia water. The mixture was stirred at room temperature for 24 hours. The precipitated crystals were separated by filtration, washed with cooled water and then dried to give 6.8 g (0.04 mol) of 2,5-dioxopiperazine-3-acetamide. The yield was 40% based on dimethyl L-aspartate.

Example 3

While stirring at ice cooled temperatures, 50 ml of methylene chloride solution containing 17.1 g (0.1 mol) of monochloroacetic acid anhydride was added to 150 ml of methylene chloride solution containing 16.1 g (0.1 mol) of dimethyl L-aspartate and 14 ml (0.1 mol) of triethylamine. After stirring for an hour at ice temperatures and for 3 hours at room temperature, the mixture was washed successively with 2N-HCl, 5% $NaHCO_3$ solution and saturated aqueous sodium chloride solution and then finally dried over anhydrous sodium sulfate. Sodium sulfate was removed by filtration and the filtrate was concentrated under reduced pressure. To the residue was added 40 ml of 28% ammonia water. The mixture was stirred at room temperature for 24 hours. The precipitated crystals were removed by filtration, washed with cooled water and then dried to give 7.0 g (0.041 mol) of 2,5-dioxopiperazine-3-acetamide. The yield was 41% based on dimethyl L-aspartate.

Example 4

After 19.8 g (0.1 mol) of dimethyl L-aspartate hydrochloride was suspended in 150 ml of chloroform, 14 ml (0.1 mol) of triethylamine was added to the suspension to neutralize. After 9.5 g (0.1 mol) of monochloroacetic acid was added to the mixture, 21.7 g (0.105 mol) of dicyclohexylcarbodiimide was added thereto while stirring at ice cooled temperatures. The mixture was stirred for an hour and then at room temperature overnight. The precipitated dicyclohexylurea was removed by filtration. After washing successively with 2N-HCl, 5% $NaHCO_3$ solution and saturated aqueous sodium chloride solution, the filtrate was dried over anhydrous sodium sulfate. Sodium sulfate was removed by filtration and the filtrate was concentrated under reduced pressure. To the residue was added 40 ml of 15% ammonia water. The mixture was stirred at room temperature for 36 hours. The precipitated crystals were removed by filtration, washed with cooled water and then dried to give 3.4 g of 2,5-dioxopiperazine-3-acetamide. The filtrate was concentrated under reduced pressure and a small quantity of water was added to the residue, which was allowed to stand in a refrigerator overnight. The precipitated crystals were filtered and dried to give 1.4 g of 2,5-dioxopiperazine-3-acetamide. Both crystals were combined. The yield was 28% based on dimethyl L-aspartate.

Example 5

After 19.8 g (0.1 mol) of dimethyl L-aspartate hydrochloride was suspended in 150 ml of toluene, 84 g of 10% $NaHCO_3$ solution was added to the suspension. After 25 g of $NaHCO_3$ was further added thereto, 11.4 g (0.1 mol) of monochloroacetyl chloride was added to the mixture while stirring. After stirring at room temperature for 5 hours, fractionation was performed and the aqueous phase was removed. The toluene layer was washed successively with 2N-HCl and 5% $NaHCO_3$ solution. Toluene was removed by distillation and 40 ml of 28% ammonia water was added to the residue. The mixture was stirred at room temperature overnight. The precipitated crystals were removed by filtration, washed with cooled water and dried to give 6.5 g of 2,5-dioxopiperazine-3-acetamide. The yield was 38% based on dimethyl L-aspartate.

Example 6

To 150 ml of chloroform solution containing 17.5 g (0.1 mol) of diethyl L-aspartate was added 9.5 g (0.1 mol) of monochloroacetic acid. The mixture was stirred. At ice cooled temperatures, 21.7 g (0.105 mol) of dicyclohexylcarbodiimide was added to the mixture followed by stirring for an hour. Thereafter the mixture was stirred at room temperature overnight. The precipitated dicyclohexylurea was removed by filtration. The filtrate was concentrated under reduced pressure and the residue was dissolved in ethyl acetate. After washing successively with 2N-HCl, 5% $NaHCO_3$ solution and saturated aqueous sodium chloride solution, the ethyl acetate layer was dried over anhydrous sodium sulfate. Sodium sulfate was removed by filtration and the filtrate was concentrated under reduced pressure. To the residue was added 30 ml of 28% ammonia water. The mixture was stirred at room temperature for 24 hours. The precipitated crystals were removed by filtration, washed with cooled water and then dried to give 6.7 g (0.039 mol) of 2,5-dioxopiperazine-3-acetamide. The yield was 39% based on dimethyl L-aspartate.

Having now fully described the invention, it will be apparent to one of ordinary skill in the art that many changes and modifications can be made thereto without departing from the spirit or scope of the invention as set forth herein.

## Claims

**1.** 2,5-Dioxopiperazine-3-acetamide.

**2.** A process for preparing 2,5-dioxopiperazine-3-acetamide which comprises:
reacting monochloroacetic acid with a dialkyl L-aspartate; and
treating the resulting dialkyl chloroacetyl-L-aspartate with ammonia.

**3.** A process for preparing 2,5-dioxopiperazine-3-acetamide which comprises:
reacting monochloroacetyl chloride with a dialkyl L-aspartate; and
treating the resulting dialkyl chloroacetyl-L-aspartate with ammonia.

**4.** A process for preparing 2,5-dioxopiperazine-3-acetamide which comprises:
reacting monochloroacetic acid anhydride with a dialkyl L-aspartate; and
treating the resulting dialkyl chloroacetyl-L-aspartate with ammonia.

**5.** The process of Claim 2, 3 or 4 wherein the amount of ammonia in the reaction medium is at least 2-fold (in molar amount) relative to dialkyl monochloroacetyl-L-aspartate.

**6.** The process of Claim 5, wherein the amount of ammonia ranges from 2 to 100 moles relative to dialkyl monochloroacetyl-L-aspartate.

European Patent Office

# EUROPEAN SEARCH REPORT

Application Number

EP 91 12 2308

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOINORGANIC CHEMISTRY<br>vol. 3, no. 3, 1974, NEW YORK<br>pages 243 - 260;<br>G.B. BRUBAKER ET AL.: 'Models for metal-protein interaction. Copper(II) complexes with some substituted cyclic dipeptides.'<br>*page 250, 258; CAS RN 52661-99-1*<br>--- | 1 | C07D241/08 |
| A,P | WO-A-9 114 378 (THE NUTRASWEET COMPANY)<br>* page 4, line 30 - page 5, line 6 *<br>* page 8 - page 10, line 20; claims 56-58; examples 1-2B,3-5B *<br>--- | 1-6 | |
| A | US-A-3 976 773 (A.C.W. CURRAN)<br>* column 2, line 48 - column 3, line 35 *<br>----- | 1-6 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 21 APRIL 1992 | BOSMA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)